# EUROPEAN PATENT APPLICATION

(11) **EP 2 332 601 A1**
(43) Date of publication of application: **15.06.2011**
(21) Application number: 09809860.1
(22) Date of filing: 24.08.2009
(51) Int. Cl.: A61M 5/28, A61M 5/31

(54) **SYRINGE**

(30) Priority: 25.08.2008 JP 2008214861
(71) Applicant: Denki Kagaku Kogyo Kabushiki Kaisha, Tokyo 103-8338 (JP); Taisei Kako Co., Ltd., Kita-ku Osaka-shi Osaka 531-0072 (JP)
(72) Inventor: TABATA Kota, Itoigawa-shi Niigata 949-0305 (JP); KOHSAKA Masanobu, Itoigawa-shi Niigata 949-0305 (JP); HIOKI Kazutoshi, Itoigawa-shi Niigata 949-0305 (JP); MATSUMOTO Ippei, Ibaraki-shi Osaka 567-0054 (JP); ASAHI Norihiko, Ibaraki-shi Osaka 567-0054 (JP)
(74) Representative: Gulde Hengelhaupt Ziebig & Schneider
(86) International application number: PCT/JP2009/064683
(87) International publication number: WO 2010/024209

(57) **Abstract**

There is provided a syringe capable of preventing the occurrence of "link-thread" from the distal end nozzle when the cap is pulled out, even in the case where the medicinal liquid filled has a high viscosity.

The inner peripheral surface (631) of the seal cap (63) in the cap (6) is brought into close contact with the outer peripheral surface (214) of the distal end side portion of the distal end nozzle (21) in the barrel (2) so that the distal end opening (211) is hermetically closed off. At the same time, the outer peripheral side of the luer lock adapter (22) is covered with the outer tubular cover (62) of the cap. The bulge part (213) is formed in the distal end side portion of the inner aperture (212) in the distal end nozzle so that the aperture diameter (*d*) of the distal end opening is reduced to a small aperture diameter and, in addition, the length of seal (s) by the seal cap (63) is made to be a relatively short, predetermined length. The diameter of the filled medicinal liquid to be sucked in until the vacuum breaks down when the cap is pulled out, is made very small and, in addition, the vacuum is made to break down instantly, thereby making it possible to prevent the occurrence of "link-thread" even if the medicinal liquid filled is of high viscosity.

## Description

### Technical Field

The present invention relates to syringes and, more particularly, relates to syringes suitable for use as pre-filled syringes filled beforehand with medicinal liquid. With regard to the shipment of pre-filled syringes, they are shipped in such a manner that their caps are mounted respectively on their distal end nozzles to thereby place the medicinal liquid filled inside is placed in a hermetically sealed state. More specifically, this invention relates to a syringe capable of getting rid of the possibility of the occurrence of "link-thread" (i.e., the formation of a viscous thread) from the distal end nozzle at the time the syringe is actually used after shipment in the form of a pre-filled syringe.

### Background Art

Generally, in a typical pre-filed syringe, the medicinal liquid filed inside is placed in a hermetically sealed state between the gasket engaged in through the proximal end opening of the pre-filled syringe and the cap mounted onto the distal end nozzle. Then, with the filled medicinal liquid in such a hermetically sealed state, the pre-filled syringe is subjected to a sterilization treatment and, thereafter, is shipped and provided to a medical institution and so on.

If performing sterilization of the pre-filled syringe by the application of heat, this causes expansion of the air in the space in the cap. To cope with this, there has been known a sterilization treatment for the purpose intended to maintain sealing performance by avoiding inconveniences in association with expansion of the air in the space in the cap. For example, Patent Literature I discloses a pre-filled syringe of the following type. That is, this disclosure shows that there is formed a luer lock so as to encircle a distal end nozzle of the pre-filled syringe, that a cap is mounted onto the distal end nozzle so as to provide also covering of the outer peripheral surface of the luer lock, and that there is formed in the outer peripheral surface of the luer lock a deaeration groove extending in the direction from the distal end to the proximal end. In a pre-filled syringe of this type, the deaeration groove prevents the air, enclosed in an empty space between the distal end nozzle and the luer lock, from expanding upon receipt of heat or the like in association with the sterilization treatment. This prevents the cap from being drawn off.

In addition, with regard to the property of sealing between the distal end nozzle and the cap, there has been known a pre-filled syringe of the type that is configured so as to secure a higher sealing property without having to employ an extra seal material (see, for example, Patent Literature 2). In this pre-filled syringe, there is formed on the side of the proximal end of the distal end nozzle a nozzle side thread engaging part, and there is formed in the distal end part of the distal end nozzle a circular thick part. In addition, there are formed in the cap a tubular part which includes a sealing part and which is mounted onto the distal end nozzle and a cap side thread engaging part which is threadedly engaged with the nozzle side thread engaging part. The tubular part of the cap is in circular contact with the circular thick part of the distal end part of the distal end nozzle while the sealing part of the cap enters the distal end opening of the distal end nozzle.

### Citation List

### Patent Literature

Patent Literature 1: JP-A-20Q2-210011
Patent Literature 2: JP-A-2004-2 83465

### Summary of Invention

### Technical Problem

However, if the filling of medicinal liquid into a pre-filled syringe is done by means of vacuum drawing and if, after the distal end nozzle is closed by the cap and enters into a hermetically closed state, the cap is removed off the distal end nozzle at the time of actual use of the pre-filled syringe, this results in "link-thread" by the medicinal liquid extending between the cap and the distal end nozzle. Therefore, it is conceivable that such "link-thread" may cause inconvenience.

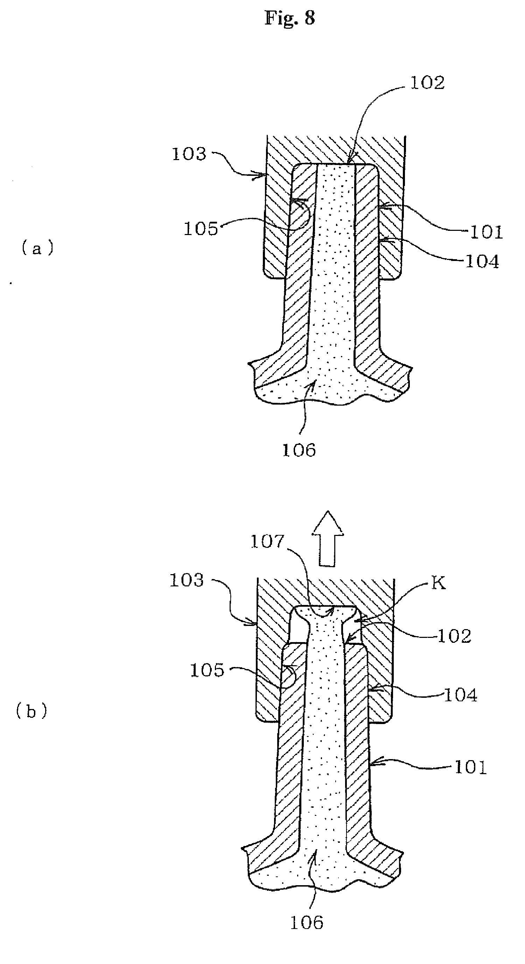

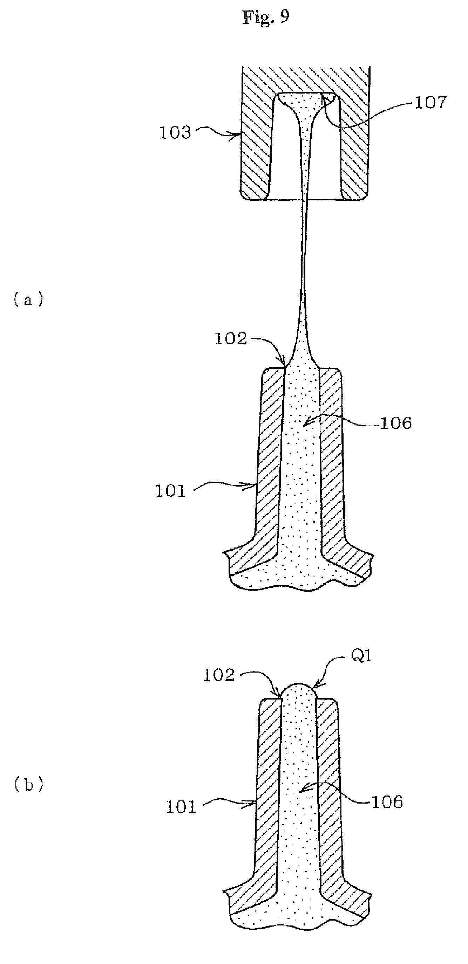

That is, in a state as exemplarily shown in Figure 8(a) in which state the distal end opening 102 of the distal end nozzle 101 is closed by the cap 103, the inner peripheral surface 105 of the cap 103 is in close contact with the outer peripheral surface 104 of the distal end nozzle 101 on the distal end side thereof whereby the medicinal liquid 106, filled inside, is held in a hermetically closed state. And when the cap 103 is pulled off of the distal end nozzle 101 in the direction indicated by an arrow of Figure 8(b) at the time of actual use of the pre-filled syringe, the medicinal liquid adhering to the cap inner back surface 107 is sucked from the distal end opening 102 of the distal end nozzle 101 with the movement of the cap 103 in the pull-off direction. The conceivable reason for this is as follows. That is, the inner peripheral surface 105 of the cap 103 moves in the pull-off direction while it remains in close contact with the outer peripheral surface 104 of the distal end nozzle 101 and, as a result, the gap space *K* defined between the inner back surface 107 of the cap 103 and the side of the distal end opening 102 of the distal end nozzle 102 expands while it remains hermetically sealed. This causes the gap space *K* to enter into a semi-vacuum state (reduced pressure state). For example, if the cap 103 is tilted with respect to the distal end nozzle 101 for the close contact between the inner peripheral surface 105 of the cap 103 and the outer peripheral surface 104 of the distal end nozzle 101 to become loose for even a moment, this allows entry of air into the gap space *K*, thereby resulting in breakdown of the semi-vacuum state as stated above. However, there may occur such "link-thread" that the medicinal liquid extends, in a threadlike manner, from the distal end nozzle 101 to the cap 103, even after the gap space *K* is released from the reduced pressure state when the cap 103 is completely detached from the distal end nozzle 101, as exemplarily shown in Figure 9(a). It is conceivable that the higher the viscosity of the filled medicinal liquid 106, the more likely that the aforesaid "link-thread" occurs and that, as the viscosity of the filed medicinal liquid 106 becomes higher, the degree of "link-thread" tends to expand.

If such "link-thread" occurs, the result is that the medicinal liquid scatters immediately after the "link-thread" is broken. This will cause inconveniences. That is, the scattered medicinal liquid may adhere to the clothes and the hands of a healthcare practitioner who is dealing with the pre-filled syringe. And, the liquid bead *Q1* (see Figure (b)) is formed by surface tension at the distal end opening 102 of the distal end nozzle 101 after the "link-thread" is broken, so that the liquid bead *Q1* is in an exposed state to the outside space.

With this situation in mind, the present invention was developed. Accordingly, an object of the present invention is to provide a syringe capable of avoiding the occurrence of "link-thread" even if the medicinal liquid filled has a high viscosity and therefore capable of avoiding the occurrence of inconvenience due to the occurrence of "link-thread".

### Solution to Problem

In order to accomplish the aforesaid object, the present invention is intended for a syringe which comprises: a synthetic resin barrel which is provided with a distal end nozzle, the distal end nozzle projecting and opening on the side of a distal end of said barrel; a cap which, when mounted onto the distal end nozzle, hermetically closes a distal end opening of the distal end nozzle; and a gasket which, when engaged in through a proximal end opening of the barrel, hermetically closes the side of a proximal end of the barrel. And this syringe has the following characteristics. That is to say, the aperture diameter of an inner aperture of the distal end nozzle, at least, at the position of the distal end opening of the distal end nozzle is set so as to fall within the aperture diameter range of substantially from 0.1 to 1.0 mm. More preferably, the aperture diameter of the inner aperture is set so as to fall within the range of from 0.5 to 1.0 mm.

For the case of the present invention, even if, in the case where the medicinal liquid is filled in the inside of the barrel to form a pre-filled syringe, suction is applied to the filled medicinal liquid adhered to the cap side in the early stage of pulling-off of the cap, the diameter of the filled medicinal liquid to be sucked in is extremely small, as corresponds to the aperture diameter of the distal end opening, since the distal end opening of the distal end nozzle is formed so as to have a small aperture diameter falling within the range of from 0.1 to 1.0 mm as described above. Consequently, even if the medicinal liquid filled is a high viscosity medicinal liquid, the medicinal liquid adhering to the cap is torn apart instantly as the cap is further pulled off, and no "link-thread" will occur. Stated another way, even in the case where a medicinal liquid of high viscosity is filled to form a pre-filled syringe, it is possible to avoid the possibility of the occurrence of "link-thread" at the point of use, i.e., when the cap is removed, thereby making it possible to prevent the occurrence of inconvenience due to the occurrence of "link-thread". Besides, the existence of a liquid bead remaining at the distal end opening of the distal end nozzle and exposed to the outside space after the cap is completely removed, is negligible, as corresponds to the aforesaid aperture diameter. Therefore, the bare possibility of the occurrence of taint damage can be avoided as much as possible. In addition, from a view point of the prevention of forming "link-thread", the better the smaller the aperture diameter, but if the aperture diameter is set so as to fall within the range of from 0.5 to 1.0 mm, this makes it possible to realize both the convenience of manufacture of the entire barrel including the nozzle by means of synthetic resin molding and the prevention of the occurrence of the aforesaid "link-thread".

In accordance with the aforesaid invention, it is possible to form a pre-filled syringe by filling the inside of the barrel, hermetically closed off by the cap mounted onto the distal end nozzle and the gasket engaged into the barrel, with a medicinal liquid whose viscosity falls within the range of from 1000 to 60000 mPa·s (the unit "mPa·s" = millipascal seconds; same in the following). In other words, by the filling of a medicinal liquid of high viscosity falling within the range of from 1000 to 60000 mPa·s, there is formed a pre-filled syringe. As such a high viscosity medicinal liquid, hyaluronic acid preparations may be used. Even in the case where pre-filled syringes filled with hyaluronic acid preparations are used, it is possible to avoid the possibility of the occurrence of "link-thread" at the point of use, i.e., when the cap is removed, thereby making it possible to prevent the occurrence of inconvenience due to the occurrence of "link-thread".

In addition, it can be arranged such that the cap includes a tubular part for engagement onto the distal end nozzle. In addition, it can be arranged such that the inner peripheral surface is extended from the opening end on one side of the tubular part to the inner back surface on the other side so that the tubular part is closed off at one end. And, when the tubular part is engaged onto the distal end nozzle from the opening end so that the cap is mounted in the state that the inner peripheral surface of the tubular part is in close contact with the outer peripheral surface of the distal end nozzle, the inner peripheral surface of the tubular part from the position of the distal end opening of the distal end nozzle to the opening end of the tubular part enters into a state of close contact to become liquid tightly sealed, with the cap mounted. The length to be sealed thus (seal length) can be set so as to fall within the range of substantially from 2.0 to 6 mm. More preferably, the seal length can be set so as to fall within the range of substantially from 3.0 to 6.0 mm. As a result of such arrangement, in combination with the aforesaid aperture diameter setting, it becomes possible to more definitely prevent the possibility of the occurrence of "link-thread" at the time of removal of the cap (i.e., at the point of use) and, at the same time, it becomes possible to definitely ensure sealability. That is, from a view point of ensuring sealability, there is required some degree of length as the seal length, but it becomes necessary that such a required length as the seal length should be made shorter when aiming to achieve early destruction of the vacuum pressure reduction tendency (as will be hereinafter described) in order to prevent the occurrence of "link-thread". For the case of the invention of claim 6, even if the gap space between the cap and the distal end nozzle lapses into a vacuum pressure reduction tendency in the early stage of movement of the cap in the pull-off direction, such tendency can be broken instantly to get rid of suction power against the medicinal liquid by setting the seal length so as to fall within the aforesaid short range of substantially from 3.0 to 6.0 mm, as described above. These make it possible to more definitely prevent the occurrence of "link-thread", even in the case where high viscosity preparations are filled as the medicinal liquid and, in addition, even if there occurs "link-thread", this "link-thread" occurrence can be eliminated substantially simultaneously with destruction of the vacuum pressure reduction tendency or early before that destruction, thereby making it possible to suppress the "link-thread" to a minimum.

In order to set the aperture diameter range in the present invention as described above, it suffices if a part or all of the inner aperture of the distal end nozzle is formed so as to have an aperture diameter that falls within the aforesaid aperture diameter range and, in the case of the latter partial diameter aperture formation in the inner aperture, the inner aperture of the distal end nozzle is reduced in diameter over the range of length from 0.3 to 3.0 mm from the position of the distal end opening to the proximal end side whereby the inner aperture of the distal end nozzle is formed so as to have an aperture diameter falling within the aforesaid aperture diameter range. If the range of the reduction in diameter is set less than 0.3 mm, this may cause a tendency of deficiency in strength against the pressure at the time the medicinal liquid filled in the barrel is pushed out. On the other hand, if the range of the reduction in diameter is set longer than 3.0 mm, this results in formation of a lengthy thick portion with the outer peripheral surface of the distal end nozzle constituting a male luer taper, thereby causing the possibility of the occurrence of sink at the time of synthetic resin molding. For the case of claim 8, the outer peripheral surface of the distal end nozzle is formed into a predetermined male luer taper by setting the range of the reduction in diameter so as to fall within the aforesaid length range and, at the same time, it becomes possible to definitely form the inner aperture of the distal end nozzle so that it has an aperture diameter capable of preventing the occurrence of "link-thread" without causing any inconvenience such as the occurrence of sink due to synthetic resin molding. That is, while realizing the requisition for the male luer taper with respect to the outer peripheral surface of the distal end nozzle, it additionally becomes possible to realize the aforesaid aperture diameter range while avoiding the formation of an excessively thick portion and the occurrence of inconvenience due to synthetic resin molding.

### Advantageous Effects Of Invention

As has been described, in accordance with the syringe of the present invention, even if, in the case where a medicinal liquid is filled in the inside of the barrel to form a pre-filled syringe, suction is applied to the filled medicinal liquid adhered to the cap side in the early stage of pulling-off of the cap, the diameter of the filled medicinal liquid to which suction is applied can be made extremely small, as corresponds to the aperture diameter of the distal end opening, by forming the distal end opening of the distal end nozzle so as to have a small aperture diameter falling within the range of from 0.1 to 1.0 mm. Consequently, even if the medicinal liquid filled is a high viscosity medicinal liquid, the medicinal liquid adhered to the cap is torn apart instantly as the cap is pulled off to a further extent, thereby making it possible to avoid the occurrence of "link-thread". That is, even in the case where a medicinal liquid of high viscosity is filled to form a pre-filled syringe, it is possible to avoid the possibility of the occurrence of "link-thread" at the point of use, i.e., when the cap is removed, thereby making it possible to prevent the occurrence of inconvenience due to the occurrence of "link-thread". Besides, it becomes possible that the existence of a liquid bead remaining at the distal end opening of the distal end nozzle in an exposed state to the outside space after the cap is completely removed, can be made negligible, a corresponds to the aforesaid aperture diameter. Therefore, the bare possibility of the occurrence of taint damage can be avoided as much as possible.

In particular, by setting the aperture diameter such that it falls within the range of from 0.5 to 1.0 mm, it becomes possible to realize both the convenience of manufacture of the entire barrel including the nozzle by means of synthetic resin molding and the prevention of the occurrence of "link-thread".

Even if formed as a pre-filled syringe whose barrel is filled with a medicinal liquid having a high viscosity of from 1000 to 60000 mPa·s, it is still possible to avoid, when using such a pre-filled syringe, the possibility of the occurrence of "link-thread" at the time of removal of the cap, thereby preventing the occurrence of inconvenience due to the occurrence of "link-thread". And, even when forming a pre-filled syringe using, as a medicinal liquid, hyaluronic acid having a high viscosity as described above, it becomes possible to avoid the possibility of the occurrence of "link-thread" at the time of removal of the cap, thereby preventing the occurrence of inconvenience due to the occurrence of "link-thread".

1n combination with the aforesaid setting of the aperture diameter, by the setting that the seal length, along which the inner peripheral surface of the tubular part enters into a state of close contact to be liquid tightly sealed in the state that the cap is mounted, is made to fall within the range of substantially from 2.0 to 6.0 mm, it becomes possible to more definitely ensure the prevention of the occurrence of "link-thread" at the time of removing the cap (i.e., at the point of use) and, at the same time, it becomes possible to definitely ensure sealability. That is, even if the gap space between the cap and the distal end nozzle lapses into a vacuum pressure reduction tendency in the early stage of movement of the cap in the pull-off direction, such tendency is broken down instantly, thereby making it possible to eliminate suction power against the medicinal liquid filled. This makes it possible to more definitely prevent the occurrence of "link-thread" even if high viscosity preparations are filled as the medicinal liquid. Here, by setting, as in claim 6, the seal length so as to fall within the range of from 3.0 to 6.0 mm from a view point of ensuring sealability, it becomes possible to obtain more definite sealability.

By forming a part or all of the inner aperture of the distal end nozzle to have an aperture diameter falling within the aforesaid aperture diameter range, it becomes possible to definitely manufacture a syringe according to the present invention. Furthermore, by forming the inner aperture of the distal end nozzle to have an aperture diameter falling within the aforesaid aperture diameter range over the range of length (from 0.3 to 3.0 mm) from the position of the distal end opening to the proximal end side, it becomes possible to prevent the formation of an excessively thick portion while realizing the requisition for a male luer taper with respect to the outer peripheral surface of the distal end nozzle. This makes it possible to definitely realize the aforesaid aperture diameter range while preventing the occurrence of inconvenience due to synthetic resin molding whereby the avoidance of "link-thread" by the present invention can be accomplished.

### Brief Description of Drawings

Figure 1 is a perspective view illustrating a syringe according to an embodiment of the present invention.
Figure 2 is an exploded perspective view of the syringe of Figure 1.
Figure 3 is a longitudinal cross-sectional view of the syringe of Figure 1.
Figure 4 is an enlarged cross-sectional view illustrating in an exploded manner a portion on the distal end side and a cap of the syringe of Figure 1.
Figure 5 is a corresponding view to Figure 4 illustrating a state where the cap of Figure 4 is mounted onto the distal end side portion of the syringe.
Figure 6(a) is a corresponding view to Figure 4 illustrating a state where the cap is slightly withdrawn away relative to the state as shown in Figure 5, and Figure 6(b) is a corresponding partial view to Figure 4 illustrating a state where the cap is completely removed from the state as shown in Figure 6(a).
Figure 7 is a table showing the test results of testing conducted to check whether there occurs "link-thread" or not.
Figure 8, comprised of Figure 8(a) and Figure 8(b), is a partially enlarged cross-sectional view for explaining the occurrence of inconvenience in a conventional technique wherein Figure 8(a) illustrates a state where the distal end nozzle is hermetically sealed by the cap and Figure 8 (b) illustrates a state where the cap is slightly withdrawn away relative to the state of Figure 8(a).
Figure 9, comprised of Figure 9(a) and Figure 9(b), is a partially enlarged cross-sectional view for explaining the occurrence of inconvenience in a conventional technique wherein Figure 9(a) illustrates a state where there occurs "link-thread" upon completion of the pulling-off of the cap from the state of Figure 8(b) and Figure 9(b) illustrates a state of the distal end nozzle after the "link-thread" is broken from the state of Figure 9(a).

### Description of Embodiments

Hereinafter, embodiments of the present invention will be described with reference to the drawings.

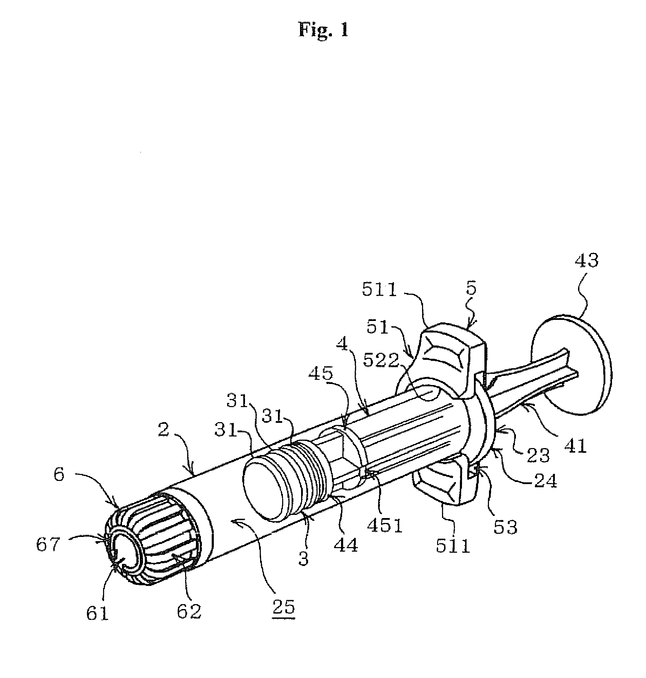

Figures 1-3 illustrate a syringe according to an embodiment of the present invention, in which figures a barrel which is a syringe tube of the syringe 1 is denoted by reference numeral 2, a gasket is denoted by reference numeral 3, a plunger rod is denoted by reference numeral 4, a finger grip is denoted by reference numeral 5 and a cap for sealing is denoted by reference numeral 6.

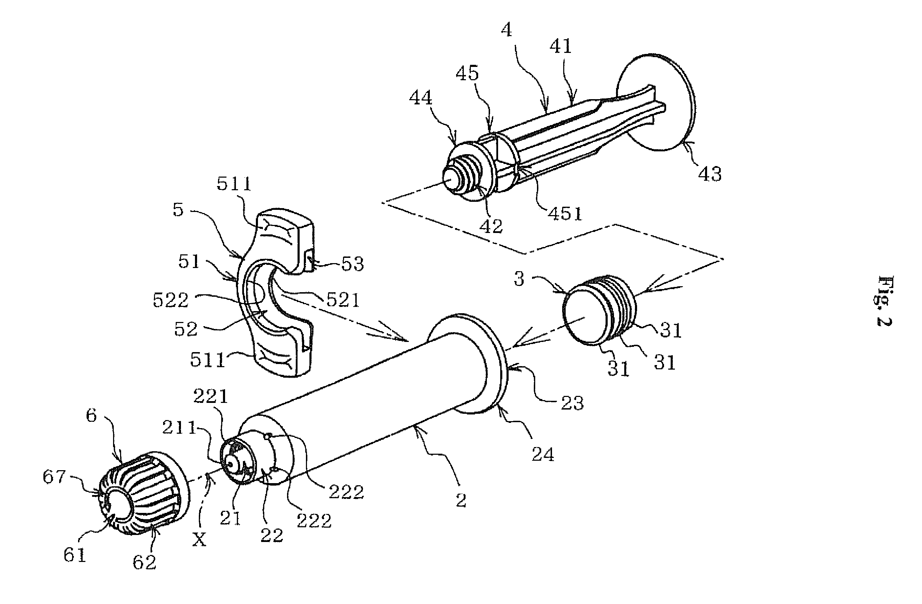

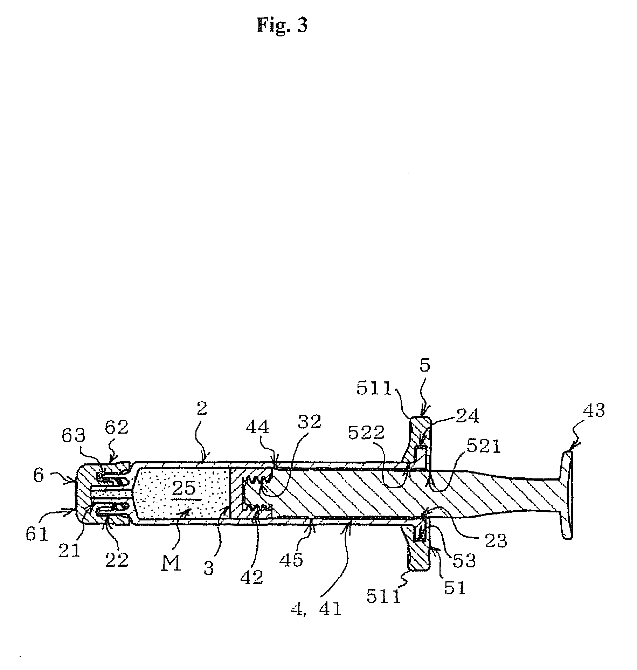

The barrel 2 is made of transparent plastic (synthetic resin). Formed on the side of a distal end of the barrel 2 are a distal end nozzle 21 and a luer lock adapter 22 (see Figure 2 and Figure 3 for both of these parts). On the other hand, formed on the side of a proximal end of the barrel 2 are a proximal end opening 23 (see Figure 3) and a proximal end flange 24 projecting on the side of an outer peripheral surface of the proximal end opening 23. And, it is configured that the cap 6 is detachably mounted onto the distal end nozzle 21 and the luer lock adapter 22 and that the finger grip 5 is detachably mounted to the proximal end flange 24 from the lateral direction. The distal end side of the barrel 2 is formed in a double tube structure by the distal end nozzle 21 and the luer lock adapter 22. And the cap 6 is mounted, in a close contact manner, onto the distal end nozzle 21 which is in fluid communication with the inside of the barrel 2, thereby liquid tightly sealing off the distal end opening of the barrel 2 while providing covering of the outer peripheral surface of the luer lock adapter 22. Details of the structure of the distal end side of the barrel 2 will be hereinafter described.

The gasket 3, made of, for example, rubber, is configured such that it is closely engaged, through the proximal end opening 23 of the barrel 2, into the barrel 2 and is slid along in the barrel 2 when pushed or pulled by the plunger rod 4 to be hereinafter described. A plurality of ribs 31, 31, ... having a slightly expanded diameter are formed integrally with and on the outer peripheral surface of the gasket 3. Each rib 31 closely and liquid tightly contacts with the inner peripheral surface of the barrel 2, thereby blocking leakage of the medicinal liquid held in the barrel 2 on the distal end side ahead of the gasket 3. In addition, there is formed in the gasket 3 a concave part 32 (see Figure 3) which opens to the proximal end side of the barrel 2 and which has in its inner peripheral surface a thread groove, and a thread part 42 (see Figure 2 and Figure 3) of the plunger rod 4 is screwed in the concave part 32 so that the gasket 3 is integrally coupled to the distal end of the plunger rod 4. With the plunger rod 4 and the gasket 3 made integral with each other, there is now constituted a plunger. And, a medicinal liquid chamber 25 (see Figure 3) for holding medicinal liquid is defined, by partitioning, in the inside of the barrel 2 ahead of the gasket 3.

The plunger rod 4 includes a rod main body 41 which is shaped like a cross in transverse cross section, a thread part 42 which projects from the distal end position of the rod main body 41 in the direction of the distal end of the barrel 2 and an operation part 43 which extends circumferentially from the proximal end position of the rod main body 41 and, in addition to these parts, the plunger rod 4 further includes a disc part 44 and a disc part 45 at the distal end position of the rod main body 41 and at at the intermediate position of the rod main body 41 at a given distance away from the distal end position in the direction of the proximal end side, respectively. The disc part 44 at the distal end position is given an outside diameter capable of abutting and covering almost the entire backside of the gasket 3. And by connecting the gasket 3 to the thread part 42, the gasket 3 is borne from the backside thereof for homogeneous transmission of a pushing force from the plunger rod 4 to the gasket 3. On the other hand, the disc part 45 is given an outside diameter which is set as follows. That is, the outside diameter of the disc part 45 is smaller, just by such a slight extent that it becomes minimum in the range that allows the disc part 45 to advancingly and retreatingly move within the barrel 2, than the inside diameter of the barrel 2. But the outside diameter of the disk part 45 is larger, by a predetermined amount, than the outside diameter of a virtual circular arc formed by connecting together outer peripheral end edges of the cross-shaped body constituting the rod main body 41. This part that projects so as to have a larger diameter than the outside diameter of the rod main body 41 abuts and stops against an aperture edge 521 of the finger grip 521 (hereinafter described), thereby preventing a further movement in the pull-off direction. This prevents the plunger rod 4 from being pulled off of the barrel 2.

In addition, a concave part 451 (see Figure 1 or Figure 2) is formed by concavely cutting away a part of the outer peripheral edge of the disc part 45 into a concave shape, and gas for sterilization is circulated, through the concave part 451, between the proximal end side and the distal end side in the barrel 2. That is, the concave part 451 establishes mutual fluid communication between a semi-hennetically closed space sandwiched between both the disc parts 44, 45 in the barrel 2 and the inside of the barrel 2 in fluid communication through the proximal end opening 23 to the outside, thereby making gas for sterilization (for example, hydrogen peroxide gas) circulatable.

The finger grip 5 has a grip main body 51 from which grip parts 511, 511 stretch out to the right-hand side and to the left-hand sides, respectively so as to project bilaterally across the proximal end flange 24 of the barrel-2, thereby forming a finger hook portion for grasping at the time of performing a syringe operation with the syringe 1. The grip main body 51 is of the type that includes an aperture part 52 into which the barrel 2 is inserted passing completely through the central position of the grip main body 51 and a concave groove part 53 which is in fluid communication with the aperture part 52 and which is able to accommodate the proximal end flange 24. And, there is an aperture edge 521 which is the aperture edge of the aperture part 52 and which is situated on the proximal end side across the concave groove part 53, the aperture edge 512 being configured such that it has an inside diameter which is equal to the outside diameter of the rod main body 41 of the plunger rod 4 and which is set to be smaller than the outside diameter of the disc part 45 and there is an aperture edge 522 on the distal end side which is configured such that it has a diameter approximately equal to the outside diameter of the barrel 2. And, by engagement of the finger grip 5 into the proximal end flange 24 of the barrel 2 from the lateral direction, the finger grip 5 becomes immobile and the proximal end opening 23 is narrowed by the aperture edge 521 on the proximal end side. This allows movement of the plunger rod 4, but when the disc part 45 of the plunger rod 4 is shifted in the pull-off direction to a position where it abuts and stops against the aperture edge 521, the plunger rod 4 is prevented (backstopped) from making a further pull-off movement in the direction of the proximal end side.

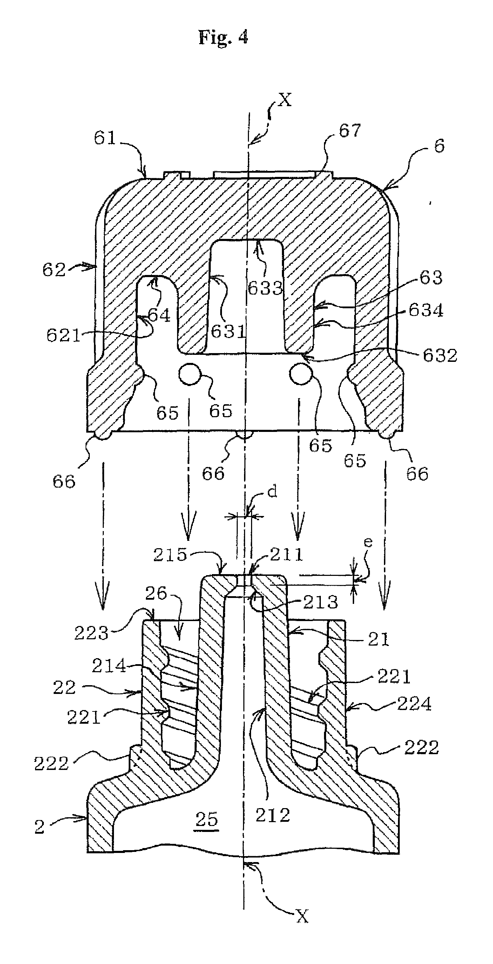

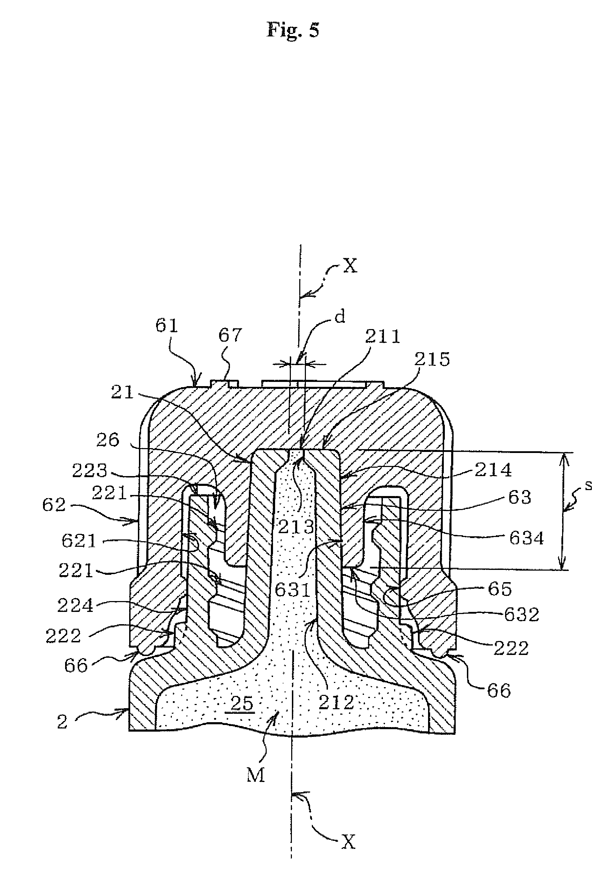

In the following, referring to Figure 4 and Figure 5, the structure of the proximal end side of the barrel 2 and the structure of the cap 6 will be described in detail.

The distal end nozzle 21 constitutes an inner tubular portion of the double tube structure. The distal end nozzle 21 is configured as follows. That is, the distal end nozzle 21 extends along the central axis *X* up to a certain position so that it projects more than the luer lock adapter 22 of the barrel 2 by a given length in the direction of the distal end side, and its outer peripheral surface 241 constitutes a male luer taper part. And, it is set such that the aperture diameter, *d*, of the distal end nozzle 21 at the position of the distal end opening 211 (see Figure 4) has a predetermined small diameter. That is, the aperture diameter *d* is formed such that it has a small diameter dimension selected from among the following aperture diameter ranges: Practically, an aperture diameter range 0.1-1.0 mm; preferably, either an aperture diameter range 0.4-1.0 mm or an aperture diameter range 0.5-1.0 mm; more preferably, either an aperture diameter range 0.4-0.7 mm or an aperture diameter range 0.5-0.7 mm. Although it can be conceivable that as the aperture diameter *d* becomes smaller, the diameter of the filled medicinal liquid sucked in when the cap 6 is pulled off becomes smaller to thereby more definitely ensure the avoidance of the occurrence of "link-thread", it is required to consider manufactural conditions. The threshold limit value for the small diameter side is subjected to the restriction mainly from the manufactural viewpoint, whereas the threshold limit value for the large diameter side is subjected to the restriction mainly from the functional viewpoint. In other words, if the distal end nozzle 21 is formed together with the barrel 2 by means of synthetic resin molding, this will accompany difficulties with stably and precisely forming the aperture diameter *d* at less than 0.1 mm and, in addition, this will further accompany difficulties with stably, precisely and mass-productively forming the aperture diameter *d* at even less than either 0.4 or 0.5 mm. On the other hand, if the aperture diameter *d* is in excess of 1.3 mm, it becomes impossible to accomplish the prevention of "link-thread" (i.e., the object of the present invention) in the case where the filled medicinal liquid exhibits a high viscosity, as will be hereinafter described. Here, with regard to the way of representation used here for the aperture diameter, it should be noted that, for example, what is meant by the representation "the aperture diameter range 0.5-0.7 mm" is an aperture diameter range of from 0.5 mm to 0.7 mm (i.e., an aperture diameter range of from not less than 0.5 mm to not more than 0.7 mm) and, in that regard, what are meant by "not less than" and "not more than" are practical ranges. For example, when represented as "not less than 5 mm", this representation includes also 0.49 mm and so on, and when represented as "not more than 7 mm", this representation includes also 0.71 mm and so on. Also in the following description, the similar range representation has the similar definition.

In order for the aperture diameter *d* to be a small diameter as described above, it is advisable to employ either one of the following ways. That is, either the inner aperture of the distal end nozzle 21 is formed such that it has the aperture diameter *d* throughout, or the entire inner aperture of the distal end nozzle 21 is formed into a taper shape in parallel with the male luer taper part so as to have the aperture diameter *d* at the position of the distal end opening 211, or the inner aperture of the distal end nozzle 21 is partially reduced so as to have the aperture diameter d, or preferably a distal end side part of the inner aperture 212 is formed (in a predetermined range) so as to have the aperture diameter *d* as exemplarily shown in the figure. When formed in the predetermined range on the distal end side (as exemplarily shown in the figure), the inner aperture 212 is reduced in diameter by forming a bulge part 213 which is situated on the distal end side of the inner aperture 212 and which bulges inwardly over a predetermined range of length e from the distal end opening 21 whereby the inside diameter of the bulge part 213 becomes equal to the the aperture diameter *d*. As the length range *e*, it suffices to set a length range of, substantially, from 0.3 mm to 3.0 mm from the position of the distal end opening 211 on the distal end side of the inner aperture 212. If the length range *e* is too short, this may cause the possibility that the bulge part 213 yields to the pressure exerted when the filled medicinal liquid is pushed out and itself undergoes folding breakage. On the other hand, if the length range e is, conversely, too long, this causes the thick portion to become bulky, as corresponds to the extension in the length range e and, as a result, sink is prone to take place. Therefore, the aforesaid length range is preferable.

The luer lock adapter 22 constitutes an outer tubular portion which encircles the outer peripheral side of the distal end nozzle 21. The luer lock adapter 22 is formed on the outer peripheral side of the distal end nozzle 21 and in a coaxial manner, relative to the central axis X, with the distal end nozzle 21 across an annular space 26 into which the female luer taper part, such as a syringe needle or the like, is placed. There are formed in the inner peripheral surface of the luer lock adapter 22 two threads 221, 221 for luer-locking of the female luer taper part. In addition, there are formed in a proximal end part on the outer peripheral side of the luer lock adapter 22 a plurality of convex parts 222, 222, ... at respective positions at a distance from each other in the circumferential direction (see also Figure 2). Each convex part 222 prevents the inner peripheral surface of an outer tubular cover part 62 of the cap 6 from being placed in close contact with an outer peripheral surface 224 of the luer lock adapter 22, as will be hereinafter described.

The cap 6 is of the type that is formed in one piece of a synthetic resin such as rubber or other like material. The cap 6 has an inner and outer double tubular structure that is opened to the proximal end side wherein the inner and outer double tubular structure of the cap 6 is formed by an outer tubular cover part 62 which extends from the periphery of a top wall part 61 on the distal end side to the proximal end side, and a cap seal part 63 which extends, on the inner peripheral side of the outer tubular cover part 62, from the inner surface of the top wall part 61 to the proximal end side so as to be arranged coaxially with the outer tubular cover part 62.

The cap seal part 63 is formed so as to include an inner peripheral surface 631 and an opening end surface 632. The inner peripheral surface 631 has a shape capable of close contact, when the cap 6 is mounted onto the distal end nozzle 21, with an outer peripheral surface 214 of the distal end nozzle 21 while being elastically compressed by a given amount in the radial direction. And, under such a close contact state, the opening end surface 632 extends for a given seal length *s* from the distal end opening 211 of the distal end nozzle 21 to the proximal end side and is positioned. The seal length *s* is set by selection from among the dimensions of a range of from 2.0 to 6.0 mm, preferably either from among the dimensions of a range of from 3.0 to 6.0 mm or from among the dimensions of a range of from 3.0 to 5.0 mm. The seal length *s* is assigned the aforesaid ranges, the reason for which is that if the seal length *s* is too short then it becomes impossible to aim at maintaining and securing the sealability and, conversely, if the seal length *s* is too long then it becomes impossible to effectively avoid the occurrence of "link-thread" in relation to the viscosity of the filled medicinal liquid even when the aperture diameter *d* of the distal end nozzle 21 is set at a dimension on the practical minimum side.

To sum up, the seal length *s* is set much shorter than that of conventional caps of the type that is brought into close contact with the outer peripheral surface 214 of the distal end nozzle 21 for hermetical sealing of the distal end opening 211 whereby the cap seal part 63 is prevented from close contact with most of the distal end nozzle 21 on the proximal end side. This is done with a view to promptly breaking the vacuum state when the cap 6 is pulled off. To this end, the length of projection of the cap seal part 63 from the top wall part 61 to the proximal end side along the central axis *X* is set much shorter than the length of projection of the distal end nozzle 21 to the distal end side.

In addition, it is preferable in the aforesaid mounted state that the setting of geometry is made for an inner back surface 633 of the seal cap part 63 and a distal end surface 215 of the distal end nozzle 21 to be placed in close contact with each other, but these surfaces may be placed in such a state that they are separated a very small dimension. The reason for this is that the sealing-off of the distal end opening 211 is secured by close contact of the amount of the seal length *s* of the inner peripheral surface 631 of the seal cap part 63 to the outer peripheral surface 214 of the distal end nozzle 21.

The cap seal part 63 is formed such that, in the aforesaid mounted state in which the cap 6 is mounted onto the distal end nozzle 21 and the seal cap part 63 is placed within the annular space 26, there is left, between the outer peripheral surface 634 and the inner peripheral surface of the luer lock adapter 22, a gap. In addition, the radial width of the annular groove 64 between the seal cap part 63 and the outer tubular cover 62 is set greater than the radial width of the luer lock adapter 22 including the thread 221 and, in addition, the dimensions and the shape thereof are set so that, in the aforesaid mounted state, gaps are formed, respectively, between the groove bottom surface of the annular groove 64 and the distal end surface 223 of the luer lock adapter 22 and between the inner peripheral surface 621 of the outer tubular cover part 62 and the outer peripheral surface 224 of the luer lock adapter 22, thereby allowing the annular space 26 to be in fluid communication with the outside even in the aforesaid mounted state. The maintaining of this fluid communication is definitely ensured by the following means. That is, for example, a plurality of hemispheroidal projections 65, 65,..., formed in the inner peripheral surface of the outer tubular cover part 62 so as to project from their respective positions spaced circumferentially apart from each other, abut and stop against the outer peripheral surface 224 of the luer lock adapter 22 in the aforesaid mounted state, and a plurality of hemispheroidal projections 66, 66,..., likewise formed in the proximal end surface of the outer tubular cover part 62 so as to project from their respective positions spaced circumferentially apart from each other, abut and stop against the surface of the barrel 2 on the distal end side in the aforesaid mounted state, thereby ensuring that the fluid communication between the annular space 26 and the outside is maintained.

Formed integrally with and on the distal end surface of the top wall part 61 is an arrow-shaped part 67 in the form of a convex (see Figure 2). The arrow-shaped part 67 is provided for the purpose of guiding and showing a user that the cap 6 can be removed easily by rotating it in the arrow direction.

Next, a brief description will be made with regard to the flow of process up to the shipping of the foregoing syringe in the form of a pre-filled syringe. The flow of process up to the shipping includes an individual sterilization step, a medicinal liquid filling/assembly step, a packaging step and a final sterilization step which steps are performed in that order. The individual sterilization step is a step in which component parts, such as the barrel 2, the gasket 3, the plunger rod 4, the finger grip 5 and the cap 6, are individually subjected to a sterilizing treatment such as, for example, a high-temperature steam sterilizing treatment. In the medicinal liquid filling/assembly step, with the cap 6 and the gasket 3 assembled to the barrel 2, the medicinal liquid chamber 25 is filled with a medicinal liquid by means of, for example, a vacuum filling method. Thereafter, the plunger rod 4 is inserted so that its distal end is coupled to the gasket 3. Then, the finger grip 5 is mounted to the flange 24 to form a pre-filled syringe (see the state shown in Figure 1). In the packaging step, the pre-filled syringe after having being filled with the medicinal liquid is placed in a package body (such as a blister container, a package bag having a gas permeable part, or the like) which is then hermetically sealed. With the syringe packed in the package body, the final sterilization step is carried out using hydrogen peroxide gas or the like.

The present embodiment is intended for using, as the medicinal liquid (pharmaceutical preparation) to be filled in the medicinal liquid filling/assembly step, medicinal liquids having a high viscosity of from 1000 to 60000 mPa·s. Such a high viscosity medicinal liquid includes hyaluronic acid preparations. As an example of the hyaluronic acid preparations, there is a solution prepared by dissolving either hyaluronate sodium derived by microbial fermentation or hyaluronate sodium derived by extraction from chicken comb, cow's eye vitreous body or umbilical cord, or the like, into injection water or injection solution liquid such as normal saline solution. And, even in the case where such a high viscosity medicinal liquid is filled to constitute a pre-filled syringe, it is still possible to avoid the occurrence of "link-thread" at the point of use (i.e., upon removal of the cap), thereby preventing the occurrence of inconvenience due to the occurrence of "link-thread".

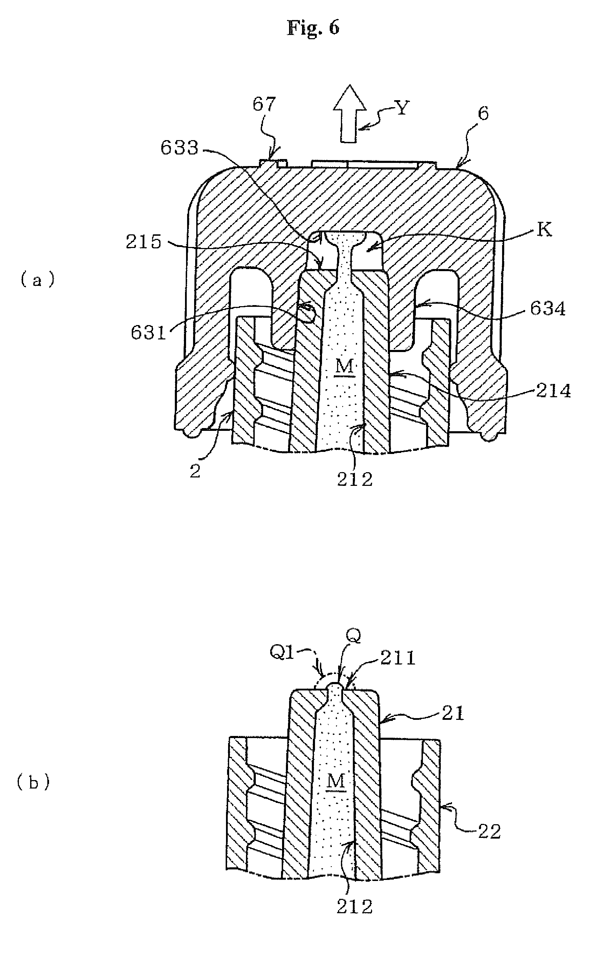

That is, as shown in Figure 6(a), when the cap 6 is withdrawn from the barrel 2 in the direction indicated by an arrow Y, a gap space *K* between the inner back surface 633 of the seal cap part 63 and the distal end surface 215 of the distal end nozzle 21 will have a vacuum pressure reduction tendency. Therefore, as the cap 6 is moved in the pull-off direction, a filled medicinal liquid *M* in a state of adhering to the inner back surface 633 of the seal cap part 63 is sucked from the distal end opening 211. In this case, however, the diameter of the filled medicinal liquid *M* sucked from the distal end opening 211 is equal to or smaller than the aperture diameter *d* of the distal end opening 211 (see Figure 4) and, therefore, disconnection tends to take place and, in addition, the amount of medicinal liquid itself drawn by suction decreases in proportion to the square of the rate of diameter reduction of the aperture diameter, in comparison with conventionally used ones. Besides, the seal length *s* of the seal cap part 63 (see Figure 5) is shorter than conventionally used ones and, therefore, the aforesaid vacuum pressure reduction tendency is instantly broken by movement of the cap 6 in the pull-off direction, thereby causing suction power against the filled medicinal liquid *M* to disappear. These make it possible to prevent "link-thread" from occurring when the cap 6 is pulled off, even in the case of using a pharmaceutical preparation having a high viscosity as the filled medicinal liquid M. In addition, even if "link-thread" occurs, such "link-thread" is got rid of at the same time that the vacuum pressure reduction tendency is broken or early before that, thereby making it possible to suppress the "link-thread" to a minimum.

In addition to the above, the size of a liquid bead *Q* (see Figure 6(b)) left at the distal end opening 211 of the distal end nozzle 21 when the cap 6 is completely removed can also be reduced to a large extent with the reduction in diameter of the distal end opening 211. That is, it also becomes possible to reduce the existence of the liquid bead *Q* to be exposed to the outside space in proportion to the square of the rate of aperture diameter reduction, in comparison with to the liquid bead *Q1* of Figure 9(b). In addition, at the time of removal of the cap 6, this removing operation of the cap 6 can be carried out easily by removing it from the barrel 2 while rotating it in the direction indicated by the arrow of the arrow-shaped part 67 and, besides, it becomes possible to slow down the appearance of a tendency of vacuum pressure reduction by preventing the gap space *K* from rapid expansion whereby the degree of suction against the filled medicinal liquid *M* can also be reduced. This make a contribution to the avoidance of the occurrence of "link-thread".

### Other Embodiments

It should be noted that the present invention is not limited to the aforesaid embodiment and, therefore, includes other various embodiments. That is, the syringe shown in the aforesaid embodiment has the luer lock adapter 22 at the distal end side of the barrel 2 and the outer tubular cover part 62 for covering the luer lock adapter 22. This is however should not be considered restrictive in any way. It is possible to form a barrel of the type without the luer lock adapter 22 and it is possible to form a cap of the type without the outer tubular cover part 62. This is because the main idea of the present invention is the configuration of the distal end nozzle 21 and the configuration of the seal cap part 63 which are shown in the aforesaid embodiment.

### Examples

Syringes in the basic form of the aforesaid embodiment were filled with high viscosity medicinal liquid and were used as testing pre-filled syringes. By variously varying the combination of the aperture diameter of the distal end opening and the seal length of the seal cap part, testing was conducted in order to check whether there occurred "link-thread" when the cap was removed.

As the barrel 2, seven different types were prepared having their respective aperture diameters *D* (namely, 1.5 mm, 1.3 mm, 1.0 mm, 0.7 mm, 0.6 mm, 0.5 mm and 0.4 mm) over the range of length e of the inner aperture's 212 distal end side from the distal end opening 211(see Figure 4). On the other hand, as the cap 6, five different types were prepared having their respective seal part lengths *S* (namely, 8.0 mm, 6.0 mm, 4.5 mm, 3.0 mm and 2.0 mm).

The number of combinations of the aperture diameter *D* and the seal length *S* is 35 (= 7 (types) x 5 (types) and 10 test pieces were prepared for each of these 35 combinations. Each test piece was filled with hyaluronic acid preparation (as a medicinal liquid to be filled) of from 30000 to 500000 mPa·s (measured by a type-B viscometer). These 350 (35 x 10) test pieces were used for testing and, more specifically, 10 test pieces per each of the 35 combinations of the aperture diameter *D* and the seal length *S* were subjected to pull-off testing. Here, the representation of *D* as the aperture diameter and the representation of *S* as the seal length indicate, respectively, the aperture diameter and the seal length in the present testing and, on the other hand, the representation of *d* as the aperture diameter and the representation of *s* as the seal length indicate, respectively, the aperture diameter and the seal length in the foregoing embodiment.

The pull-off (removal) testing was carried out in the following way. Each pre-filled syringe was fixed to a tension-compression testing device, with its cap positioned downside. Then, the cap was withdrawn downwardly by means of a special jig at a speed of 1000 mm/min. The existence or nonexistent of the occurrence of "link-thread" was checked with eyes. Visual confirmation of the occurrence of "link-thread" was classified as the presence of "link-thread". On the other hand, when the occurrence of "link-thread" was not visually confirmed, this was classified as the absence of "link-thread". The arrangement that the cap was positioned downside was made on the assumption that "link-thread" was most likely to take place in such positioning, in other words, on the assumption of the worst possible use status.

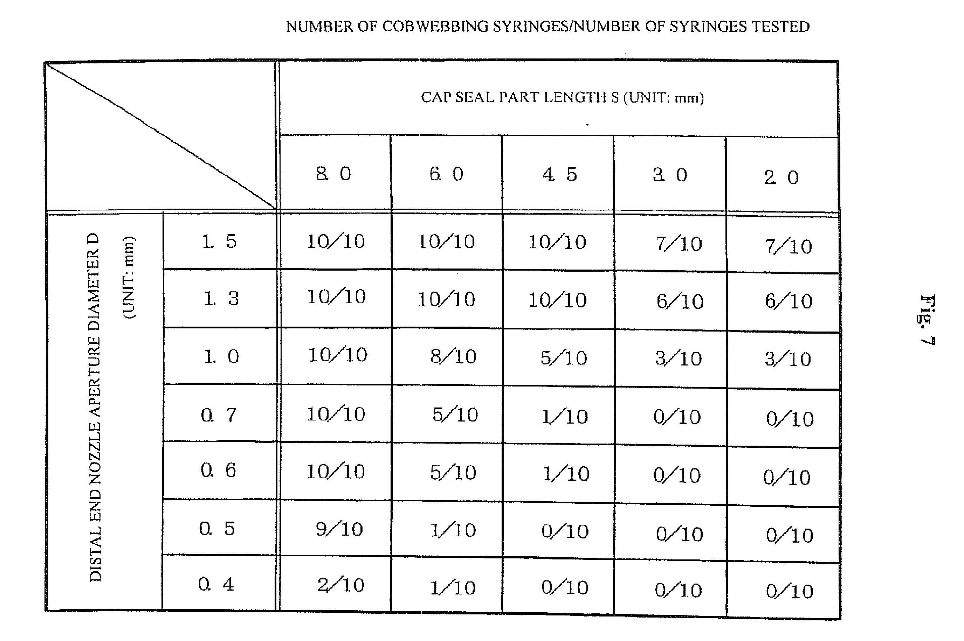

Figure 7 in the form of a table summarizes the results of the testing. Here, in the notation of *n*/*N* (both *n* and *N* are integral numbers), *n* is indicative of the number of syringes that had undergone "link-thread", and *N* is indicative of the number of syringes which were tested under the same conditions (10 syringes in the present testing example), and *n*/*N* is indicative of the ratio of the number of syringes that had undergone "link-thread" against all of the 10 syringes. For example, the notation of 1/10 shows that, of all the 10 syringes tested, the number of syringes that had undergone "link-thread" is one, and that the remaining 9 syringes were free from "link-thread".

The following particulars were derived from the testing results. That is, when the aperture diameter *D* = 0.4 mm, there occurred no "link-thread" to any of the seal lengths *S* of 2.0 mm, 3.0 mm and 4.5 mm and, even in the case where the seal length *S* = 6.0 mm, the occurrence of "link-thread" remained infrequent that only a single syringe out of all the ten syringes had undergone "link-thread". Moreover, even in the case where the seal length S= 8.0 mm, the occurrence of "link-thread" still remained infrequent that only two syringes out of all the ten syringes had undergone "link-thread". Accordingly, it is conceivable that the occurrence of "link-thread" should be avoided effectively, regardless of the combination with the seal length S, if the aperture diameter *d* (see Figure 4) is selected from among diameters falling within the range of from not more than 0.4 mm to the manufacturable minimum.

As in the case where the aperture diameter *D* = 0.4 mm, when the aperture diameter *D* = 0.5 mm, there occurred no "link-thread" to any of the seal lengths *S* of 2.0 mm, 3.0 mm and 4.5 mm and, even in the case where the seal length *S* = 6.0 mm, the occurrence of "link-thread" remained infrequent that only a single syringe out of all the ten syringes had undergone "link-thread". However, in the case where the seal length *S* = 8.0 mm, the occurrence of "link-thread" took a sudden jump that nine syringes out of all the ten syringes had undergone "link-thread". Consequently, in the case where the aperture diameter *D* = 0.5 mm, it is conceivable that there is a clear boundary between the seal length *S* of 6.0 mm and the seal length *S* of 8.0 mm from a point of view of the avoidance of the occurrence of "link-thread". Accordingly, it is conceivable that, when choosing an aperture diameter of 0.5 mm as the aperture diameter *d* (see Figure 4), the occurrence of "link-thread" is effectively avoided by choosing an aperture diameter/seal length combination in which the aperture diameter *d* is 0.5 mm and the seal length *s* is either in the range of substantially not more than 7.0 mm nor less than 2.0 mm or in the range of substantially not more than 6.0 mm nor less than 2.0 mm.

When the aperture diameter *D* = either 0.6 or 0.7 mm, there occurred no "link-thread" to any of the seal lengths *S* of 2.0 mm and 3.0 mm and in the case where the seal length *S* = 4.5 mm, the occurrence of "link-thread" remained infrequent that only a single syringe out of all the ten syringes had undergone "link-thread". However, in the case where the seal length *S* = 6.0 mm, the occurrence of "link-thread" took a jump that five syringes out of all the ten syringes had undergone "link-thread". And in the case where the seal length *S* = 8.0 mm, "link-thread" had occurred in all of the ten syringes. Consequently, when the aperture diameter *D* = either 0.6 or 0.7 mm, there is a clear boundary around the seal length *S* of 6.0 mm from a point of view of the avoidance of the occurrence of "link-thread" and, to be on the safe side, it is conceivable that the aforesaid boundary is preferably set at the seal length *S* of between 4.5 and 6.0 mm (for example, 5.0 mm). Accordingly, it is conceivable that, when choosing an aperture diameter of either 0.6 or 0.7 mm as the aperture diameter *d* (see Figure 4), the occurrence of "link-thread" is effectively avoided by choosing an aperture diameter/seal length combination in which combination the aperture diameter *d* is either 0.6 or 0.7 mm and the seal length *s* falls within the range of substantially not more than 5.0 mm nor less than 2.0 mm.

When the aperture diameter *D* = 1.0 mm, there occurred "link-thread" in three of all the ten syringes in both the case where the seal length *S* = 2.0 mm and the case where the seal length *S* = 3.0 mm. When the seal length *S* = 4.5 mm, there occurred "link-thread" in five of all the ten syringes. When the seal length S= 6.0, the frequency of the occurrence of "link-thread" increased that eight of all the ten syringes had undergone "link-thread". When the seal length *S* = 8.0 mm, all of the ten syringes had undergone "link-thread". Accordingly, it is conceivable that, when choosing an aperture diameter of 1.0 mm as the aperture diameter *d* (see Figure 4), the seal length *s* to be combined therewith is required to fall within the range of substantially not more than 3.0 mm nor less than 2.0 mm in order that the occurrence of "link-thread" is avoided.

Furthermore, when the aperture diameter *D* = 1.3 mm, there occurred "link-thread" in six of all the ten syringes in both the case where the seal length *S* = 2.0 mm and the case where the seal length *S* = 3.0 mm. When the seal length *S* = 4.5 mm, all of the ten syringes had undergone "link-thread". Accordingly, it is conceivable that, in order to avoid the occurrence of "link-thread", the selecting of dimensions of substantially not more than 1.0 mm as the aperture diameter *d* (see Figure 4) is required to make.

The present invention provides a syringe which is distributed and provided as a pre-filled syringe in the filed of medicine or other like field.

### Reference Signs List

- 2: barrel
- 3: gasket
- 6: cap
- 21: distal end nozzle
- 23: proximal end opening
- 25: medicinal liquid filling chamber
- 63: seal cap part (tubular part)
- 211: distal end nozzle's distal end opening
- 212: distal end nozzle's inner aperture
- 213: distal end nozzle's bulge part
- 214: distal end nozzle's outer peripheral surface
- 631: seal cap part's inner peripheral surface
- 632: seal cap part's opening end surface (opening end)
- 633: seal cap part's inner back surface
- *d*: aperture diameter
- s: seal length

## Claims

1. A syringe comprising:
a synthetic resin barrel which is provided with a distal end nozzle, said distal end nozzle projecting and opening on the side of a distal end of said barrel;
a cap which, when mounted onto said distal end nozzle, hermetically closes a distal end opening of said distal end nozzle; and
a gasket which, when engaged in through a proximal end opening of said barrel,
hermetically closes the side of a proximal end of said barrel;
wherein the aperture diameter of an inner aperture of said distal end nozzle, at least, at the position of said distal end opening of said distal end nozzle is set so as to fall within the aperture diameter range of substantially from 0.1 to 1.0 mm.

2. The syringe as set forth in claim 1,
wherein a medicinal liquid having a viscosity that falls within the range of from 1000 to 60000 mPa·s is filled in the inside of said barrel hermetically closed by said cap mounted onto said distal end nozzle and said gasket engaged into said barrel.

3. The syringe as set forth in claim 2,
wherein a part or all of said inner aperture of said distal end nozzle is formed so as to have an aperture diameter that falls within the aforesaid aperture diameter range.

4. The syringe as set forth in claim 3,
wherein said inner aperture of said distal end nozzle is reduced in diameter over the range of length from 0.3 to 3.0 mm from the position of said distal end opening to the proximal end side whereby said inner aperture of said distal end nozzle is formed so as to have an aperture diameter that falls within the aforesaid aperture diameter range.

5. The syringe as set forth in claim 2,
said cap including a tubular part for engagement onto said distal end nozzle wherein an inner peripheral surface of said tubular part extends from an opening end of said tubular part on one side to an inner back surface of said tubular part on the other side so that said tubular part is closed off at one end and wherein said tubular part is engaged onto said distal end nozzle from said opening end so that said cap is mounted, with said inner peripheral surface of said tubular part in close contact with an outer peripheral surface of said distal end nozzle,
wherein the length of seal, along which liquid tight sealing is achieved when, with said cap mounted, said inner peripheral surface of said tubular part from the position of said distal end opening of said distal end nozzle to said opening end of said tubular part enters into a state of close contact, is set so as to fall within the range of substantially from 2.0 to 6.0 mm.

6. The syringe as set forth in claim 5,
wherein a part or all of said inner aperture of said distal end nozzle is formed so as to have an aperture diameter that falls within the aforesaid aperture diameter range.

7. The syringe as set forth in claim 6,
wherein said inner aperture of said distal end nozzle is reduced in diameter over the range of length from 0.3 to 3.0 mm from the position of said distal end opening to the proximal end side whereby said inner aperture of said distal end nozzle is formed so as to have an aperture diameter that falls within the aforesaid aperture diameter range.

8. The syringe as set forth in claim 5,
wherein said seal length is set so as to fall within the range of substantially from 3.0 to 6.0 mm.

9. The syringe as set forth in claim 8,
wherein a part or all of said inner aperture of said distal end nozzle is formed so as to have an aperture diameter that falls within the aforesaid aperture diameter range.

10. The syringe as set forth in claim 9,
wherein said inner aperture of said distal end nozzle is reduced in diameter over the range of length from 0.3 to 3.0 mm from the position of said distal end opening to the proximal end side whereby said inner aperture of said distal end nozzle is formed so as to have an aperture diameter that falls within the aforesaid aperture diameter range.

11. The syringe as set forth in claim 1,
wherein the aperture diameter of said inner aperture is set so as to fall within the range of from 0.5 to 1.0 mm.

12. The syringe as set forth in claim 11,
wherein a medicinal liquid having a viscosity that falls within the range of from 1000 to 60000 mPa·s is filled in the inside of said barrel hermetically closed by said cap mounted onto said distal end nozzle and said gasket engaged into said barrel.

13. The syringe as set forth in claim 12,
wherein a part or all of said inner aperture of said distal end nozzle is formed so as to have an aperture diameter that falls within the aforesaid aperture diameter range.

14. The syringe as set forth in claim 13,
wherein said inner aperture of said distal end nozzle is reduced in diameter over the range of length from 0.3 to 3.0 mm from the position of said distal end opening to the proximal end side whereby said inner aperture of said distal end nozzle is formed so as to have an aperture diameter that falls within the aforesaid aperture diameter range.

15. The syringe as set forth in claim 12,
said cap including a tubular part for engagement onto said distal end nozzle wherein an inner peripheral surface of said tubular part extends from an opening end of said tubular part on one side to an inner back surface of said tubular part on the other side so that said tubular part is closed off at one end and wherein said tubular part is engaged onto said distal end nozzle from said opening end so that said cap is mounted, with said inner peripheral surface of said tubular part in close contact with an outer peripheral surface of said distal end nozzle,
wherein the length of seal, along which liquid tight sealing is achieved when, with said cap mounted, said inner peripheral surface of said tubular part from the position of said distal end opening of said distal end nozzle to said opening end of said tubular part enters into a state of close contact, is set so as to fall within the range of substantially from 2.0 to 6.0 mm.

16. The syringe as set forth in claim 15,
wherein a part or all of said inner aperture of said distal end nozzle is formed so as to have an aperture diameter that falls within the aforesaid aperture diameter range.

17. The syringe as set forth in claim 16,
wherein said inner aperture of said distal end nozzle is reduced in diameter over the range of length from 0.3 to 3.0 mm from the position of said distal end opening to the proximal end side whereby said inner aperture of said distal end nozzle is formed so as to have an aperture diameter that falls within the aforesaid aperture diameter range.

18. The syringe as set forth in claim 15,
wherein said seal length is set so as to fall within the range of substantially from 3.0 to 6.0 mm.

19. The syringe as set forth in claim 18,
wherein a part or all of said inner aperture of said distal end nozzle is formed so as to have an aperture diameter that falls within the aforesaid aperture diameter range.

20. The syringe as set forth in claim 19,
wherein said inner aperture of said distal end nozzle is reduced in diameter over the range of length from 0.3 to 3.0 mm from the position of said distal end opening to the proximal end side whereby said inner aperture of said distal end nozzle is formed so as to have an aperture diameter that falls within the aforesaid aperture diameter range.

21. The syringe as set forth either in any one of claims 2-10 or as set forth in any one of claims 12-20,
wherein said medicinal liquid is a hyaluronic acid preparation.

22. The syringe as set forth in either claim 1 or claim 11
wherein a part or all of said inner aperture of said distal end nozzle is formed so as to have an aperture diameter that falls within the aforesaid aperture diameter range.

23. The syringe as set forth in claim 22,
wherein said inner aperture of said distal end nozzle is reduced in diameter over the range of length from 0.3 to 3.0 mm from the position of said distal end opening to the proximal end side whereby said inner aperture of said distal end nozzle is formed so as to have an aperture diameter that falls within the aforesaid aperture diameter range.

24. The syringe as set forth in either claim 1 or claim 11,
said cap including a tubular part for engagement onto said distal end nozzle wherein an inner peripheral surface of said tubular part extends from an opening end of said tubular part on one side to an inner back surface of said tubular part on the other side so that said tubular part is closed off at one end and wherein said tubular part is engaged onto said distal end nozzle from said opening end so that said cap is mounted, with said inner peripheral surface of said tubular part in close contact with an outer peripheral surface of said distal end nozzle,
wherein the length of seal, along which liquid tight sealing is achieved when, with said cap mounted, said inner peripheral surface of said tubular part from the position of said distal end opening of said distal end nozzle to said opening end of said tubular part enters into a state of close contact, is set so as to fall within the range of substantially from 2.0 to 6.0 mm.

25. The syringe as set forth in claim 24,
wherein a part or all of said inner aperture of said distal end nozzle is formed so as to have an aperture diameter that falls within the aforesaid aperture diameter range.

26. The syringe as set forth in claim 25,
wherein said inner aperture of said distal end nozzle is reduced in diameter over the range of length from 0.3 to 3.0 mm from the position of said distal end opening to the proximal end side whereby said inner aperture of said distal end nozzle is formed so as to have an aperture diameter that falls within the aforesaid aperture diameter range.

27. The syringe as set forth in claim 24,
wherein said seal length is set so as to fall within the range of substantially from 3.0 to 6.0 mm.

28. The syringe as set forth in claim 27,
wherein a part or all of said inner aperture of said distal end nozzle is formed so as to have an aperture diameter that falls within the aforesaid aperture diameter range.

29. The syringe as set forth in claim 28,
wherein said inner aperture of said distal end nozzle is reduced in diameter over the range of length from 0.3 to 3.0 mm from the position of said distal end opening to the proximal end side whereby said inner aperture of said distal end nozzle is formed so as to have an aperture diameter that falls within the aforesaid aperture diameter range.
